Europäisches Patentamt

European Patent Office

Office européen des brevets

(11) Veröffentlichungsnummer: **0 278 224 B1**

(12) **EUROPÄISCHE PATENTSCHRIFT**

(45) Veröffentlichungstag der Patentschrift: **15.04.92**

(21) Anmeldenummer: **88100215.8**

(22) Anmeldetag: **09.01.88**

(51) Int. Cl.5: **C11D 17/00**, A61L 2/00, C11D 7/42

(54) **Verfahren zur Herstellung von desinfizierend wirkenden Kontaktlinsen-Reinigungsmitteltabletten.**

(30) Priorität: **16.01.87 DE 3701129**

(43) Veröffentlichungstag der Anmeldung:
**17.08.88 Patentblatt 88/33**

(45) Bekanntmachung des Hinweises auf die
Patenterteilung:
**15.04.92 Patentblatt 92/16**

(84) Benannte Vertragsstaaten:
**AT BE CH DE ES FR GB GR IT LI LU NL SE**

(56) Entgegenhaltungen:
**EP-A- 0 082 798**
**DE-A- 3 329 922**
**GB-A- 2 117 534**
**US-A- 3 884 826**

(73) Patentinhaber: **Henkel Kommanditgesellschaft
auf Aktien
Postfach 1100 Henkelstrasse 67
W-4000 Düsseldorf-Holthausen(DE)**

(72) Erfinder: **Kruse, Hans
Am Hallenbad 44
W-4052 Korschenbroich(DE)**
Erfinder: **Jacobs, Jochen, Dr.
Am Acker 20
W-5600 Wuppertal 1(DE)**
Erfinder: **Wisotzki, Klaus-Dieter, Dr.
Wahnenmühle 4
W-4006 Erkrath 2(DE)**
Erfinder: **Thul, Jutta
Molzhausweg 43
W-4010 Hilden(DE)**

## Beschreibung

Die zunehmende Zahl an Trägern von weichen oder harten Kontaktlinsen macht die Bereitstellung eines in jedem Falle bequem und sicher anwendbaren Systems von Desinfektions- und Reinigungsmitteln erforderlich. Hierzu werden vorwiegend Peroxoverbindungen benutzt, die Keime und Pilze zerstören, deren Überschüsse jedoch selbst wieder zerstört werden müssen, bevor die Linsen wieder auf die Pupille gesetzt werden.

So wird in der deutschen Offenlegungsschrift 28 35 652 zum Desinfizieren und Reinigen von Kontaktlinsen ein Redoxsystem bispielsweise aus Ascorbinsäure und in wäßriger Lösung stark alkalisch reagierendem Alkalipercarbonat verwendet, in dessen wäßrige Lösung die Kontaktlinsen etwa 5 Minuten lang hineingelegt werden. Nach dabei erfolgter Desinfektion werden die Linsen gegebenenfalls mit Kochsalzlösung gespült. Dann können sie wieder auf die Augen aufgebracht werden. Das Redoxsystem soll trocken vermischt und als Pulver, Granulat oder Tablettenform feuchtigkeitsdicht verpackt angeboten werden. Über Art und Herstellung von Tabletten wird nichts ausgesagt.

Nach der deutschen Offenlegungsschrift 33 29 922 werden Kontaktlinsen dadurch desinfiziert und gereinigt, daß man sie 10 bis 20 Minuten lang in einer Lösung aus Kochsalz und einer darin gelösten Tablette (A) aus Harnstoffperoxohydrat lagert, sie anschließend 15 Minuten lang in einer frischen Lösung aus Kochsalz und einer darin gelösten Tablette (B) aus Natriumascorbat oder einer Mischung aus Ascorbinsäure und Natriumcarbonat beläßt und schließlich noch mindestens 5 Minuten lang in einer reinen Kochsalzlösung lagert. Danach können die Linsen wieder getragen werden. Es werden also für den gesamten Reinigungsvorgang zwei verschiedene Tabletten verwendet.

In der europäischen Patentanmeldung 82 798 wird ein Verfahren zum Desinfizieren und Reinigen von Kontaktlinsen beschrieben, wonach diese zunächst etwa 20 Minuten lang in eine Wasserstoffperoxidlösung gelegt werden, der anschließend zur Zersetzung von $H_2O_2$-Überschüssen eine Katalasetablette zugefügt wird. Das Enzym wirkt innerhalb von 5 Minuten. Auch hier werden die beiden erforderlichen Mittel des Systems getrennt angeboten.

Als am bequemsten für den Anwender hat sich bisher offenbar ein Desinfektions- und Reinigungsmittelsystem erwiesen, bei dem beispielsweise Harnstoffperoxohydrat in einer Kochsalzlösung zusammen mit einem kationischen, nichtionischen oder vorzugsweise amphoteren bzw. anionischen Tensid und einem Katalysator für die nachträgliche Zerstörung des Peroxidüberschusses angewendet wird. Ein solches System ist in der amerikanischen Patentschrift 4 414 127 beschrieben. Die ebenfalls gesondert verpackten Systembestandteile, nämlich zum einen das Harnstoffperoxohydrat und zum anderen eine Lösung aus dem Tensid, dem Katalysator und dem Kochsalz, werden unmittelbar vor dem Gebrauch zusammengegeben. Danach braucht sich der Anwender um nichts mehr zu kümmern.

In der nicht vorveröffentlichten europäischen Patentanmeldung 87110664.7 (EP-A-0 255 041) wird beschrieben, daß man zu ebenfalls gleichzeitig und bequem anwendbaren Desinfektions- und Reinigungsmittelsystemen für harte und weiche Kontaktlinsen mit einem Gehalt an festen Peroxoverbindungen, Reduktionsmitteln und/oder Katalysatoren und Tensiden kommt, wenn der peroxoverbindungshaltige Anteil einen Zusatz an einem Alkylglucosid, und der $H_2O_2$-neutralisierende Anteil Puffersalze zur Einstellung von pH 7 aufweist. Die einzelnen Bestandteile können auch gemeinsam zu Tabletten verpreßt werden, wenn man die miteinander unverträglichen weil reagierenden Substanzen durch "Versiegelung" mit wasserlöslichen filmbildenden Polymeren voneinander trennt und für luft- und feuchtigkeitsdichte Verpackungen sorgt.

Aus der internationalen Patentanmeldung WO86/05695 sind feste Desinfektionsmittel für Kontaktlinsen bekannt, die einen Wasserstoffperoxid liefernden Bestandteil und einen nach der Anwendung überschüssig verbleibendes Wasserstoffperoxid inaktivierenden Bestandteil enthalten, wobei letzterer mit einem Polymerenüberzug versehen ist, der eine verzögerte Auflösung des Inaktivierungsmittels bewirkt. Außerdem enthalten diese Desinfektionsmittel einen Farbindikator, der den Fortschritt der Inaktivierung anzeigen soll. Die Reinigungswirkung derartiger Desinfektionsmittel hat sich jedoch als unzureichend erwiesen.

Im Grunde ist daher das Problem einer geeigneten, sicheren und bequemen Angebotsform von desinfizierenden Reinigungsmitteln für Kontaktlinsen als bisher ungelöst anzusehen, weil es schwierig ist, temperaturempfindliche und/oder chemisch miteinander reagierende Substanzen zu kombinieren und gemeinsam zu verpacken.

Aufgabe der Erfindung war daher die Herstellung einer sogenannten Manteltablette, bei der die Außenschicht (der Mantel) aus der desinfizierenden Reinigungsmittelmischung besteht, die sich in Wasser zuerst auflöst und auf die in die Lösung hineingelegten Kontaktlinsen einwirkt. Mit zeitlicher Verzögerung soll sich dann ein sogenannter Kern der ursprünglichen Tablette lösen, der überschüssiges Desinfektions- und Reinigungsmittel neutralisiert.

Die vorliegende Erfindung betrifft nun ein Verfahren zur Herstellung von desinfizierend wirkenden Kontaktlinsen-Reinigungsmitteltabletten, daß dadurch gekennzeichnet ist, daß man in einer ersten Verfahrensstufe eine lagerstabile, tablettierbare Kernmischung, die ein Neutralisationsmittel zum Zerstören überschüssigen Wasserstoffperoxids und Einstellen der Reinigungsmittellösung auf einem pH-Wert von ca.7 darstellt, bereitet, in einer zweiten Verfahrensstufe daraus eine Kerntablette herstellt, diese in einer dritten Verfahrensstufe mit einer Lackschicht umhüllt, in einer vierten Verfahrensstufe eine Mantelmischung, die aus dem eigentlichen Reinigungsmittel auf Basis einer $H_2O_2$ liefernden Peroxoverbindung besteht, herstellt und in einer fünften Verfahrensstufe die Kerntablette in die Mantelmischung einpreßt.

Bei Anwendung dieser Tablette werden die Wirkstoffe in der gewünschten Reihenfolge freigesetzt. Bei Herstellung und Lagerung treten praktisch keine Aktivitätsverluste durch vorzeitige unerwünschte Reaktionen ein und die gereinigten Kontaktlinsen verursachen keine Reizungen am Auge, was als Zeichen der vollständigen Neutralisation des desinfizierenden Reinigungsmittels zu werten ist.

Als Bestandteile für die Mantelmischung, die desinfizierend und reinigend wirken soll, also das eigentliche Reinigungsmittel darstellt, kommen in Analogie zu den in der europäischen Patentanmeldung 87110664.7 (EP-A-0 255 041) genannten Bestandteilen Wasserstoffperoxid liefernde Verbindungen in Betracht, denen Alkylglucoside zugesetzt werden.

Als Wasserstoffperoxid bzw. $H_2O_2$-liefernde Peroxoverbindungen können z.B. Kaliumpersulfat, Melaminperhydrat und vorzugsweise Harnstoffperoxohydrat, die in wäßriger Lösung sauer reagieren, eingesetzt werden.

Als Alkylglucoside kommen solche der allgemeinen Summenformel $R_1O(C_nH_{2n}O)_y$ $(Z)_x$ in Betracht, wobei $R_1$ eine Alkylkette mit 8 - 18, vorzugsweise 12 - 14 C-Atomen, n 2 oder 3, y 0 bis 10, vorzugsweise 0, Z Glucose und x 1 bis 10, vorzugsweise 1 bis 5 bedeuten.

Diese Alkylglucoside, die selbst oberflächenaktiv sind, können vorzugsweise auch mit weiteren verträglichen Tensiden kombiniert werden, wobei mit "verträglichen Tensiden" solche nichtionische Tenside gemeint sind, die die leistungssteigernden antimikrobiellen Eigenschaften nicht rückgängig machen, z. B. Fettalkoholethoxylate, Addukte von Ethylenoxid und Propylenoxid an Fettalkohole oder n-alkyl-endgruppenverschlossene Fettalkoholethoxylate. Mit diesen Tensiden werden in der Hauptsache der Trübungspunkt der Reinigungs- und Desinfektionslösung gesteuert sowie gegebenenfalls deren schaumdämpfende Eigenschaften ausgenutzt.

Falls erforderlich, können zur Schaumdämpfung auch andere Hilfsstoffe in die Tablette integriert werden, z. B. Dimethylpolysiloxane oder deren Modifizierungen, Paraffine oder hydrophobierte Kieselsäuren.

Besonders geeignet ist ein Polysiloxan EH 7566® der Firma Goldschmidt. In Abhängigkeit vom Tensidgehalt der Reinigungslösung liegen die bevorzugten Einsatzkonzentrationen zwischen 0,01 und 0,0001 Gew.-% in der Lösung.

Bei Einsatz dieser schaumdrückenden Zusätze kann gegebenenfalls auf den Einsatzverträglicher nichtionischer Tenside neben dem Alkylglucosid verzichtet werden.

Bevorzugt werden dem peroxoverbindungshaltigen Anteil des Desinfektions-und Reinigungsmittelsystems auch noch weitere Hilfsstoffe zugesetzt, vor allem solche, die den pH-Wert während der Reinigung auf ca. pH 2 bis 7, vorzugsweise ca. pH 3,5 einstellen, wie insbesondere Zitronensäure, Salicylsäure oder Milchsäure oder Gemische davon. Diese Hilfsstoffe können die antimikrobielle Aktivität der Lösung zusätzlich deutlich steigern.

Die Alkylglucoside werden mit den pulverförmigen Peroxoverbindungen mechanisch und in bekannter Weise gemischt, wobei das Verhältnis der mittleren Molmassen von Peroxoverbindung zu Alkylglucosid $1 . 10^2 : 1$ bis $32 . 10^4 : 1$, vorzugsweise $1 . 10^3 : 1$ bis $32 . 10^3 : 1$ und insbesondere $1 . 10^3 : 1$ bis $8,3 . 10^3 : 1$ beträgt. Ein Zusatz von Kochsalz verbessert die Löslichkeit der Mantelschicht der Tablette. Mit dieser Mantelschichtmischung wird später die Kerntablette umhüllt.

Die beim Desinfektions- und Reinigungsvorgang nicht verbrauchten $H_2O_2$-Mengen werden durch die zeitlich leicht verzögerte Auflösung der das Neutralisationsmittel aus Reduktionsmittel und/oder Katalysatoren enthaltenen Kerntablette neutralisiert. Geeignete Reduktionsmittel sind z. B. Ascorbinsäure, Natriumascorbat oder Glucose. Als Katalysatoren werden Enzyme eingesetzt. Ein besonders geeignetes und bevorzugtes Enzym ist Katalase. Die Aktivität der Katalase wird in Units/mg angegeben. Eine Sigma-Unit baut, ausgehend von einer Konzentration von 10,3 $\mu$mol/ml in der Reaktionsmischung, bei pH 7 und 25 °C in 1 Minute 1 $\mu$mol $H_2O_2$ ab. Die Bestimmung erfolgt über die Messung der Absorption bei 240 nm.

Wenn man nur mit Reduktionsmittel arbeitet, benötigt man davon mindestens äquimolare Mengen in bezug auf die Peroxoverbindung. Dabei kann sich allerdings bei der Neutralisation zuviel Wärme entwickeln, die unter Umständen linsenschädigend wirken kann.

Arbeitet man nur mit Katalysatoren, d. h. Enzymen, so geht die Neutralisation des restlichen $H_2O_2$ zwar schnell aber nur bei Raumtemperatur vor sich und die Vorteile einer Wärmeentwicklung entfallen. Deshalb hat sich die Kombination eines Reduktionsmittels mit Katalase als günstig für die Neutralisation erwiesen, weil die Reinigungslösung dann bei einsetzender Neutralisation schwach erwärmt wird und Wärme dabei den zeitlichen Ablauf und die Reinigungswirkung z. B. in bezug auf die Fetentfernung günstig unterstützt.

Die Menge an Reduktionsmittel entspricht bei alleinigem Einsatz der der eingesetzten Peroxoverbindung, d. h. es werden äquimolare Mengen mit einem Überschuß von 1 bis 5, vorzugsweise 2 bis 4 Mol-% an Reduktionsmittel eingesetzt. Bei gemeinsamem Einsatz mit einem Enzym kann die Menge des Reduktionsmittels auf etwa die Hälfte bis auf etwa ein Viertel reduziert werden. Das Enzym wird je nach Aktivität in Mengen von 0,001 bis 0,2 mg/ml Lösung, vorzugsweise von 0,005 bis 0,1 mg/ml Lösung eingesetzt, bezogen auf die Menge der Peroxoverbindung.

Neben dem Reduktionsmittel und dem Enzym enthält das Neutralisationsmittel für die Reduktion der $H_2O_2$-Reste des Desinfektions-und Reinigungsmittelsystems gleichzeitig noch Puffersalze, die den pH-Wert der Gesamtlösung auf ca. pH 7 einstellen, wie Natriumhydrogencarbonat, Natriumcarbonat oder Natriumcitrat. Es kann nach Bedarf zusätzlich noch die schon angesprochenen verträglichen Tenside und/oder zusätzliche Salze wie z. B. ebenfalls Natriumchlorid und/oder Farbstoffe enthalten.

Da einerseits verhindert werden muß, daß das Reduktionsmittel und/oder das Enzym nicht vorzeitig, insbesondere nicht schon bei der Lagerung mit der Peroxoverbindung der Mantelschicht reagiert, sondern da andererseits auch das Enzym in der Kernmischung in Gegenwart von anderen Kernbestandteilen, insbeson dere Natriumhydrogencarbonat, bei der Lagerung seine Aktivität verliert, sollte nicht nur die Kerntablette mit einer wasserlöslichen Lackschicht versiegelt, sondern desgleichen auch der Enzym-oder der übrige Anteil der Kernmischung mit einer sauer oder neutral eingestellten Lackschicht umhüllt werden.

Als wasserlösliche, eine Lackschicht auf den jeweiligen Substraten bildende Substanzen kommen Polyvinylalkohol, Polywachse, Polyvinylpyrrolidon, Celluloseether und Derivate und bevorzugt Polyacrylate in Betracht. Diese Lackbildner können sowohl aus wäßriger als auch aus nichtwäßriger Lösung bzw. Lösungsmittelgemischen auf die Kermischung und/oder die Kerntablette aufgebracht werden. Bei Verwendung nichtwäßriger Lösungsmittel bzw. Lösungsmittelgemische sollen diese den Siedepunkt von Wasser bei Normaldruck nicht übersteigen Es muß soviel an Lackbildern angewendet werden, daß die Umhüllung lückenlos ist. Hierfür reichen Mengen von etwa 3 bis 15 Gew.-%, bezogen auf das zu umhüllende Substrat, im allgemeinen aus.

Da Reaktionen der Wirksubstanzen untereinander auch durch Restwassergehalte begünstigt werden, können zusätzlich wasserbindende Salze in die Tablette eingearbeitet werden, z. B. Magnesiumsulfat oder Natriumsulfat. Ihre Mengen betragen bis zu 15, vorzugsweise 5 bis 10 Gew.-%, bezogen auf das Gesamtgewicht der Tablette.

Auch der Zusatz bekannter Tablettierhilfsmittel erwies sich als zweckmäßig. Als besonders geeignet erwies sich das physiologisch verträgliche Polyethylenglykol mit einem mittleren Molgewicht von 6000. Die Anwendungsmengen betragen 1 bis 5, vorzugsweise 2 bis 3 Gew.-%, bezogen auf die zu verpressende Mischung. Es hat sich gezeigt, daß ein Vorgemisch aus Polyethylenglykol mit der Katalase zu einer genaueren Dosierbarkeit des Enzyms und zu besonders trägerstabilen Tabletten führt.

Um eine beginnende Reaktion der Mantelmasse unter Wasserfreisetzung bei der Lagerung zu verhindern, ist es angebracht, die Tabletten bei reduzierter Luftfeuchte, vorzugsweise unter etwa 15 % relativer Luftfeuchtigkeit zu verpressen (klimatisierte Räume) und zusammen mit bekannten Trockenmitteln, z. B. speziell für solche Zwekke handelsübliche Trockentabletten, oder unter Luftfeuchteausschluß zu lagern.

Es sei noch bemerkt, daß unter "Neutralisieren" im anmeldungsgemäßen Sinne die Zerstörung von überschüssigem $H_2O_2$ sowie die Einstellung der Reinigungsmittellösung auf ca. pH 7 verstanden wird. Die hierfür verwendeten Mittel sind als "Neutralisationsmittel" bezeichnet worden. Dieser Begriff wird auch nachfolgend übernommen.

Weitere Einzelheiten sind dem nachfolgenden Beispiel zu entnehmen.

## Beispiele

### Beispiel 1

Zusammensetzung der Mantelmischung:
91,86 Gew.-% Harnstoffperoxohydrat
0,06 Gew.-% Alkylglucosid aus $C_{12}/C_{14}$ Fettalkohol mit Z = 1,4
0,12 Gew.-% Umsetzungsprodukt aus $C_{12}/C_{14}$ Fettalkohol verethert mit n-Butanol und 9 Mol Ethylenoxid
7,96 Gew.-% Zitronensäure
bildeten 100 % Wirksubstanz, die durch Zusatz von 3 Gew.-% Kochsalz auf 97 % Wirksubstanz reduziert werden. Pro Tablette enthielt die Mantelmischung 1,63 g Wirksubstanz.
Zusammensetzung der Kernmischung:
0,10 Gew.-% Katalase (10 000 - 25 000 Units/mg nach Sigma)
60,08 Gew.-% Natriumascorbat
39,53 Gew.-% Natriumhydrogencarbonat
0,30 Gew.-% Umsetzungsprodukt aus einem $C_{12}$-$C_{18}$-Fettalkohol und 9 Mol Ethylenoxid (Eumulgin B®)
bildeten 100 % Wirksubstanz, die mit zusätzlich 10 Gew.-% Magnesiumsulfat verschnitten wurden. Außerdem wurden noch ca. 6 Gew.-% Polyacrylat, berechnet als Festsubstanz, und 5 Gew.-% Polyethylenglykol mit einem mittleren Molgewicht von 6000 als Tablettierhilfsmittel zugesetzt. Die Kernmischung enthielt pro Tablette 0,63 g Masse mit einem Wirksubstanzgehalt von etwa 80 bis 90 %.

Die Tabletten wurden wie folgt hergestellt:

## 1. Herstellung einer lagerstabilen, tablettierbaren Kernmischung

Durchgeführte Versuchsreihen hatten gezeigt, daß die Katalase in Gegenwart von Natriumhydrogencarbonat an Aktivität verliert. Daher wurde zunächst eine Umhüllung des Natriumhydrogencarbonates oder des katalasefreien Kerngemisches mit einer dichten, unter Anwendungsbedingungen löslichen Lackschicht erforderlich. Sie erfolgte in einer mit Heißluft betriebenen Wirbelschicht, wobei in die aufgewirbelten Substanzen mittels Zweistoffdüse 6 Gew.-%, gerechnet als Festsubstanz, einer wäßrigen und/oder lösungsmittelhaltigen, neutral eingestellten Acrylharzlösung (Copolymerisat auf Basis Polyacrylsäure und Polyacrylsäureester; "Eudragit L 30 D") aufgedüst werden. Die 6,4 %ige Acrylharzlösung wurde noch mit einem Weichmacherzusatz, nämlich 1,3 % Triethylcitrat und 1,45 % Natriumhydroxid versehen.

Ein noch besserer Überzug und eine spätere bessere Verpreßbarkeit wurde durch Einsatz gröberer Rohstoffqualitäten erreicht. Aus diesem Grund kann es zweckmäßig sein, als Ausgangsprodukte grobe käufliche, oder z. B. walzenkompaktierte Granulate einzusetzen. Die Abmischung der bedüsten Substanz bzw. Substanzen mit den Restkomponenten erfolgte in einem schonenden Mischsystem. Als Tablettierhilfsmittel dienten 3 Gew.-% Polyethylenglykol mit mittlerem Molgewicht 6000, das mit dem Enzym vermischt wurde. Ein Zusatz von 10 Gew.-% MgS0$_4$ als Trockenmittel erfolgte vorsorglich zur weiteren Verbesserung der Lagereigenschaften der Tablette.

## 2. Herstellung der Kerntablette

Die Verpressung des unter 1. angegebenen zu tablettierenden Gemisches erfolgte auf einer Rundläufer-Tablettenpresse, vorzugsweise bei reduzierter Luftfeuchte. Die hergestellten Tabletten hatten Linsenform mit einem Durchmesser von 10 mm und einer Höhe von ca. 6,5 mm. Durch empirische Optimierung des Preßdruckes, der etwa 5 t/cm$^2$ betrug, wurden stabile, eine weitere Behandlung überstehende Tabletten erhalten.

## 3. Umhüllung der Kerntabletten

Um später eine Reaktion der sauren Bestandteile des Mantels mit den alkalischen Bestandteilen des Kerns auszuschließen, ist auch eine Umhüllung der Kerntablette selbst, ebenfalls mit einer neutral eingestellten wäßrigen und/oder lösungsmittelhaltigen Acrylharzlösung, z. B. "Eudragit L 30 D", erforderlich. Dies geschah in einer sich drehenden Dragiertrommel, vorzugsweise ebenfalls in klimatisierten Räumen. Die Harzlösung wurde mittels einer Zweistoffdüse auf die in Bewegung gehaltenen Tabletten aufgedüst. Gleichzeitig wurde durch Einblasen von beispielsweise etwa 40 °C warmer Luft das mit eingedüste Wasser verdampft. Die Zugabegeschwindigkeit der Lösung wurde so mit dem Luftstrom abgestimmt, daß das eingedüste Wasser und/oder Lösungsmittel bzw. Lösungsmittelgemisch immer direkt verdampfte und die Tabletten somit nicht aneinander kleben blieben. Ein Lackauftrag (Trockensubstanz) von 3 mg/cm$^2$, entsprechend 1,8 bis 2 Gew.-% war ausreichend.

## 4. Herstellung der Mantelmischung

Um eine gleichmäßige Gemischzusammensetzung zu erhalten, mußte eine Vormischung der Komponenten mit geringen Mengen eines Teils des Harnstoffperoxohydrates, etwa 5 Gew.-%, erfolgen. Anschließend wurde dieses Vorgemisch mit den restlichen Anteilen an Harnstoffperoxohydrat und Zitronensäure sowie einem Zusatz von 3 Gew.-% Kochsalz vermischt. Der Kochsalzzusatz verbesserte die Löslichkeit der Tablette und die physiologische Verträglichkeit der gereinigten Kontaktlinsen mit dem Auge.

## 5. Ummantelung der umhüllten Kerntablette

Diese erfolgte, ebenfalls bevorzugt in klimatisierten Räumen, nach folgendem Prinzip: Die wie unter 1 bis 3 beschriebenen umhüllten Kerntabletten wurden über einen Vibrationstopf und eine Übergabescheibe in die Matrizenbohrung auf einer speziellen Rundläufer-Tablettenpresse zur Herstellung von Manteltabletten vom Fabrikat Kilian "Typ RUD" abgelegt, in die vorher ein Teil der Mantelschicht dosiert wurde. Durch das Anfahren des Oberstempels wurde eine Zentrierung der Kerntablette in der Mantelmasse erreicht, bevor der Rest der Mantelschicht aufdosiert wurde. Anschließend erfolgte die Verpressung, wobei eine einwandfreie Bindung der Mantelmasse mit dem Kern durch eine genaue empirische Abstimmung des Preßdruckes sichergestellt wurde.

Die hergestellte Tablette hatte einen Durchmesser von 16 mm und eine Höhe von 10 bis 11 mm. Auch nach Lagerung (mit Trockentabletten aus Silicagel) bei Raumtemperatur wurde kein Aktivitätsverlust festgestellt.

Nach dem gleichen Prinzip wurden Tabletten aus folgenden Zusammensetzungen hergestellt:

Beispiel 2

Zusammensetzung der Mantelmischung:
91,97 Gew.-% Harnstoffperoxohydrat
0,06 Gew.-% Alkylglucosid ($C_{12}/C_{14}$ Fettalkohol mit Z = 1,4) und
7,97 Gew.-% Zitronensäure
bildeten 100 % Wirksubstanz, die durch Zusatz von 3 Gew.-% Kochsalz auf 97 % Wirksubstanz reduziert wurden. Pro Tablette enthielt die Mantelmischung 1,63 g Wirksubstanz.

Zusammensetzung der Kernmischung:
0,10 Gew.-% Katalase (10 000 - 25 000 Units/mg nach Sigma)
60,24 Gew.-% Natriumascorbat
39,63 Gew.-% Natriumhydrogencarbonat und
0,03 Gew.-% Siloxan EH 7566®
bildeten 100 % Wirksubstanz, die noch mit ca. 6 Gew.-% Polyacrylat, berechnet als Festsubstanz und ca. 3 Gew.-% an Polyethylenglycol mit einem mittleren Molgewicht von 6000 als Tablettierhilfsmittel versetzt wurden.

Die Kernmischung enthielt pro Tablette 0,63 g Masse.

Beispiel 3

Zusammensetzung der Mantelmischung:
0,7 Gew.-% Ethylendiamintetraessigsäure Na-Salz
90,15 Gew.-% Melaminperhydrat
0,07 Gew.-% Alkylglucosid $C_{12}/C_{14}$ Fettalkohol mit Z = 1,4
9,08 Gew.-% Zitronensäure
bildeten 100 % Wirksubstanz, die durch Zusatz von 3 Gew.-% Kochsalz auf 97 % Wirksubstanz reduziert wurden. Pro Tablette enthielt die Mantelmischung 1,43 g Wirksubstanz.

Zusammensetzung der Kernmischung:
0,44 Gew.-% Katalase
99,52 Gew.-% Natriumhydrogencarbonat
0,04 Gew.-% Siloxan EH 7566®
bildeten 100 % Wirksubstanz. Außerdem wurden noch ca. 6 Gew.-% Polyvinylpyrrolidon, berechnet als Festsubstanz und ca. 3 Gew.-% an Polyethylenglycol mit einem mittleren Molgewicht von 6000 als Tablettierhilfsmittel zugesetzt.

Die Kernmischung enthielt pro Tablette 0,26 g Masse.

Die Tabletten wurden bei der Anwendung in 10 ml einer isotonischen Kochsalzlösung gelöst und die zu reinigenden Kontaktlinsen wurden etwa 20 bis 40 Minuten darin belassen. Danach war der Reinigungsprozeß abgeschlossen und die Linsen wieder tragbar. Sofern es nicht zu eisenhaltig ist, können die Tabletten auch in normalem Leitungswasser gelöst werden. Bei eisenhaltigem Wasser müssen Tabletten verwendet werden, die Komplexbildner in der Mantelmischung enthalten.

**Patentansprüche**

1. Verfahren zur Herstellung von desinfizierend wirkenden Kontaktlinsen-Reinigungsmitteltabletten, dadurch gekennzeichnet, daß man in einer ersten Verfahrensstufe eine lagerstabile, tablettierbare Kernmischung, die ein Neutralisationsmittel zum Zerstören überschüssigen Wasserstoffperoxids und Einstellen der Reinigungsmittellösung auf einem pH-Wert von ca. 7 darstellt, bereitet, in einer zweiten Verfahrensstufe daraus eine Kerntablette herstellt, diese in einer dritten Verfahrensstufe mit einer Lackschicht umhüllt, in einer vierten Verfahrensstufe eine Mantelmischung, die aus dem eigentlichen Reinigungsmittel auf Basis einer $H_2O_2$ liefernden Peroxoverbindung besteht, herstellt und in einer fünften Verfahrensstufe die Kerntablette in die Mantelmischung einpreßt.

2. Verfahren nach Anspruch 1, dadurch gekennzeichnet, daß man die Kernmischung aus Katalase, Tablettierhilfsmittel und Natriumhydrogencarbonat sowie gegebenenfalls weiteren Substanzen durch Umhüllen von Natriumhydrogencarbonat und den gegebenenfalls weiteren Substanzen mit einer dichten unter den Anwendungsbedingungen löslichen sauer oder neutral eingestellten Lackschicht umhüllt.

3. Verfahren nach Anspruch 1 und 2, dadurch gekennzeichnet, daß man die Katalase mit dem Tablettierhilfsmittel mischt.

4. Verfahren nach Anspruch 3, dadurch gekennzeichnet, daß man als Tablettierhilfsmittel Polyethylenglykol mit einem mittleren Molgewicht von 6000 verwendet.

5. Verfahren nach Anspruch 1 bis 4, dadurch gekennzeichnet, daß man die Kernmischung unter etwa 5 t/cm² zu Kerntabletten verpreßt.

6. Verfahren nach Anspruch 1 bis 5, dadurch gekennzeichnet, daß man die Kerntabletten gegebenen falls in einer Dragiertrommel mit einer dichten, unter Anwendungsbedingungen löslichen Lackschicht umhüllt.

7. Verfahren nach Anspruch 1, dadurch gekennzeichnet, daß man eine Mantelmischung aus einer Peroxoverbindung und einer organischen Säure, die die Anwendungslösung auf ca. pH 2 bis 7, vorzugsweise ca. pH 3,5 einstellt, sowie sonstigen Hilfsstoffen herstellt.

8. Verfahren nach Anspruch 1 bis 7, dadurch gekennzeichnet, daß man der Mantelmischung Alkylglucoside der Formel $R_1O(C_nH_{2n}O)_y(Z)_x$ zusetzt, wobei $R_1$ eine Alkylkette mit 8 - 18, vorzugsweise 12 - 14 C-Atomen, n 2 oder 3, y 0 bis 10, vorzugsweise O, Z Glucose und x 1 bis 10, vorzugsweise 1 bis 5 bedeutet.

9. Verfahren nach Anspruch 1 bis 8, dadurch gekennzeichnet, daß man einem Teil der Mantelschicht in eine Matrizenbohrung einer Tablettenpresse gibt, die Kerntablette hineingibt, den Rest der Mantelschicht zugibt und anschließend zu einer Manteltablette verpreßt.

10. Verfahren nach Anspruch 1 bis 9, dadurch gekennzeichnet, daß man es in klimatisierten Räumen durchführt.

## Claims

1. A process for the production of disinfecting cleaning tablets for contact lenses, characterized in that a storable, tablettable core mixture in the form of a neutralizing agent for eliminating excess hydrogen peroxide and adjusting the cleaning solution to a pH value of approx. 7 is prepared in a first process step, a core tablet is produced from this mixture in a second process step and is coated with a layer of lacquer in a third process step, a shell mixture consisting of the actual cleaning preparation based on an $H_2O_2$-yielding peroxo compound is prepared in a fourth process step and the core tablet is incorporated in the shell mixture under pressure in a fifth process step.

2. A process as claimed in claim 1, characterized in that the core mixture of catalase, tabletting aid and sodium hydrogen carbonate and, optionally, other substances is coated by coating the sodium hydrogen carbonate and the other substances, if any, with a dense, acidic or neutral lacquer layer which is soluble under the in-use conditions.

3. A process as claimed in claims 1 and 2, characterized in that the catalase is mixed with the tabletting aid.

4. A process as claimed in claim 3, characterized in that polyethylene glycol having an average molecular weight of 6,000 is used as the tabletting aid.

5. A process as claimed in claims 1 to 4, characterized in that the core mixture is compressed to core tablets under a pressure of about 5 t/cm$^2$

6. A process as claimed in claims 1 to 5, characterized in that the core tablets are coated, optionally in a coating drum, with a dense lacquer layer soluble under the in-use conditions.

7. A process as claimed in claim 1, characterized in that a shell mixture is prepared from a peroxo compound and an organic acid, which adjusts the in-use solution to approx. pH 2 to 7 and preferably to approx. pH 3.5, and other auxiliaries.

8. A process as claimed in claims 1 to 7, characterized in that alkyl glucosides corresponding to the formula $R_1O(C_nH_{2n}O)_y(Z)_x$, in which $R_1$ is an alkyl chain containing 8 to 18 and preferably 12 to 14 carbon atoms, n is 2 or 3, y is 0 to 10, preferably 0, Z is glucose and x is 1 to 10 and preferably 1 to 5, are added to the shell mixture.

9. A process as claimed in claims 1 to 8, characterized in that part of the shell mixture is introduced into a cavity bore of a tabletting press, the core tablet is introduced, the rest of the shell layer is added and the whole is pressed to form a shell tablet.

10. A process as claimed in claims 1 to 9, characterized in that it is carried out in air-conditioned rooms.

## Revendications

1. Procédé de fabrication de tablettes d'agent de nettoyage à effet désinfectant pour des lentilles de contact caractérisé en ce qu'on prépare,au cours d'une première étape du procédé, un mélange de noyau stable au stockage et pouvant être transformé en tablettes, qui constitue un agent de neutralisation pour la destruction du peroxyde d'hydrogène en excès et le réglage à environ 7 de la valeur du pH de la solution d'agent de nettoyage que, dans une deuxième étape du procédé, on utilise ce mélange pour la préparation d'un noyau de tablette, qu'on enveloppe celui-ci d'une couche de vernis au cours d'une troisième étape du procédé que, dans une quatrième étape du procédé,on prépare un mélange d'enveloppe qui est constitué de l'agent de nettoyage proprement dit à base d'un composé peroxo fournissant du

$H_2O_2$ et que, au cours d'une cinquième étape du procédé, on enfonce les noyaux de tablette dans le mélange de l'enveloppe.

2. Procédé selon la revendication 1, caractérisé en ce qu'on enveloppe le mélange du noyau constitué de catalase, d'adjuvant de pastillage et d'hydrogénocarbonate de sodium, ainsi que éventuellement d'autres substances, en enrobant l'hydrogénate de carbonate de sodium et les autres substances éventuelles avec une couche de vernis étanche, soluble dans les conditions d'utilisation et à réaction acide ou neutre.

3. Procédé selon les revendications 1 et 2, caractérisé en ce qu'on mélange la catalase à l'adjuvant de pastillage.

4. Procédé selon la revendication 3, caractérisé en ce qu'on utilise comme adjuvant de pastillage du polyéthylèneglycol ayant un poids moléculaire moyen de 6.000

5. Procédé selon les revendication 1 à 4, caractérisé en ce qu'on comprime le mélange de noyau sous une pression d'environ 5 tonnes/$cm^2$ pour former les noyaux de tablettes.

6. Procédé selon les revendications 1 à 5, caractérisé en ce qu'on enveloppe les noyaux de tablette éventuellement dans un tambour à former les dragées, avec une couche de vernis étanche et soluble dans les conditions d'utilisation.

7. Procédé selon la revendication 1, caractérisé en ce qu'on prépare un mélange d'enveloppe avec un composé peroxo et un acide organique qui permet de régler la valeur du pH de la solution d'utilisation entre environ 2 et 7 et de préférence à environ 3,5, en y ajoutant également d'autres substances auxiliaires.

8. Procédé selon les revendications 1 à 7, caractérisé en ce qu'on ajoute au mélange de l'enveloppe des alkylglucosides de la formule $R_1O(C_nH_{2n}O)_y(Z)_x$, dans laquelle $R_2$ représente une chaîne alkyle avec 8 à 18 et de préférence 12 à 14 atomes de carbone, n est égal à 2 ou 3, y est égal à un nombre compris entre 0 et 10 et de préférence est égal à 0, Z représente du glucose et x représente un nombre compris entre 1 et 10 et de préférence entre 1 et 5.

9. Procédé selon les revendications 1 à 8, caractérisé en ce qu'on introduit une partie de la couche d'enveloppe dans un alésage de matrice d'une presse a pastiller, qu'on y ajoute le noyau de la tablette, qu'on y introduit le reste de la couche d'enveloppe et qu'on le comprime ensuite pour former une tablette à enveloppe.

10. Procédé selon les revendications 1 à 9, caractérisé en ce qu'on le met en oeuvre dans des locaux climatisés.